# EUROPEAN PATENT APPLICATION

(11) **EP 1 876 235 A1**
(43) Date of publication of application: **09.01.2008**
(21) Application number: 06730175.4
(22) Date of filing: 28.03.2006
(51) Int. Cl.: C12N 15/09, C12N 1/15, C12N 1/19, C12N 1/21, C12N 5/10, C12N 9/12, C12P 19/32

(54) **NOVEL PANTOTHENATE KINASE GENE AND USE THEREOF**

(30) Priority: 29.03.2005 JP 2005094183
(71) Applicant: Kaneka Corporation, Kita-ku Osaka-shi, Osaka 530-8288 (JP)
(72) Inventor: KATO, Tomohisa, Osaka, 5670887 (JP); IKENAKA, Yasuhiro, Hyogo, 6512242 (JP)
(74) Representative: Behnisch, Werner
(86) International application number: PCT/JP2006/306227
(87) International publication number: WO 2006/104124

(57) **Abstract**

The present invention provides: a polypeptide, having a pantothenate kinase activity, which is derived from a *Corynebacterium* microorganism highly capable of producing a pantothenic acid derivative; DNA encoding the polypeptide; a recombinant vector having the DNA introduced therein; and a transformant having the DNA or the recombinant vector introduced therein. The present invention makes it possible to produce pantothenic acid derivatives such as coenzyme A in large quantities by a biological method, thereby making it possible to provide safe and high-quality pantothenic acid derivatives that are demanded in applications such as medicines, functional foods, and the like.

## Description

### TECHNICAL FIELD

The present invention relates to a gene, derived from a microorganism, which encodes an enzyme involved in the biosynthesis of coenzyme A, a transformed cell having the gene introduced therein, and a method for preparing a pantothenic acid derivative with use of the transformed cell.

### BACKGROUND ART

Coenzyme A is a type of pantothenic acid derivative, produced by almost all organisms, which is known to act as a coenzyme in several tens of types of *in vivo* reactions.

A known example of a method for producing coenzyme A is a biological method for culturing various organisms, or especially a highly productive microorganism such as bacteria and yeast and then extracting coenzyme A from a microorganism cell of the microorganism. This method is still used as a method for producing coenzyme A on a reagent scale. However, such a microorganism cell contains only a slight amount of coenzyme A. Therefore, mass production of coenzyme A requires a large number of such microorganism cells. This causes an extract operation to be cumbersome and complicated.

In addition to such extracting methods, a method for producing a cell reaction with use of a microorganism or an object obtained by processing a microorganism cell of the microorganism has been considered (Patent Document 1) (Non-patent Document 1), and was actually in practical use. However, the method for producing a cell reaction is no longer in practical use, for example, because the method is so complicated that it is difficult to set conditions, because the reaction by which pantothenic acid is phosphorylated by pantothenate kinase is rate-limiting, and because the enzyme activity is not sufficient. Further, a method using a processed microorganism cell (Patent Document 2) was not in a situation to enable stable mass production, for example, because the method uses expensive ATP.
Patent Document 1: *Tokukaisho* 60-98992
Patent Document 2: Tokukosho 49-13994
Non-patent Document 1: Shimizu S., et al., Appl. Environ. Microbiol., 48, 1118-1122 (1984)

### DISCLOSURE OF INVENTION

### PROBLEMS TO BE SOLVED BY THE INVENTION

Pantothenic acid derivatives such as coenzyme A are now being more and more widely used. Not only do they act as medicines, but also they are expected to bring about an effect as cosmetics and functional foods. Therefore, it is desired that efficient mass production of safe pantothenic acid derivatives be achieved. Especially for the purpose of use as functional foods, such a production system is important that can stably supply safe pantothenic acid derivatives. In this respect, it is considered that production of pantothenic acid derivatives by microorganisms is excellent. However, as described above, it is difficult for the conventional manufacturing methods to achieve efficient and stable mass production.

In view of these circumstances, the present invention provides a biological method that enables mass production of pantothenic acid derivatives such as coenzyme A.

### MEANS TO SOLVE THE PROBLEMS

In order to acquire, from a strain belonging to *Corynebacterium ammoniagenes* that exhibits the best reactivity in a cell reaction method, pantothenate kinase (CoaA) genes serving as rate-limiting enzymes for biosynthesis, the inventors searched a database for known genes derived from related *Corynebacterium* bacteria, and then compared sequences of the genes. As a result, the inventors discovered highly conserved regions. Focusing attention on sequences of the highly conserved regions, the inventors designed a mix primer to contain those sequences of the highly conserved regions, and then performed PCR by using genomic DNA of *Corynebacterium ammoniagenes* as a template. As a result, the inventors found an amplified fragment considered as a CoaA gene fragment. The inventors determined a DNA base sequence of the amplified fragment. As a result, the inventors obtained a sequence represented by SEQ ID NO: 1 in the

### sequence listing.

Production of a pantothenic acid derivative can be enhanced by incorporating the obtained gene into an expression vector and then by transforming, into an appropriate host, the expression vector having the obtained gene.

As described above, the present invention has a plurality of subject matters. Examples of part of the subject matters include the following:
(1) A polypeptide (A) or (B):
   (A) a polypeptide containing an amino acid sequence represented by SEQ ID NO: 2; or
   (B) a polypeptide having (i) an amino acid sequence, represented by SEQ ID NO: 2, one or more amino acids of which are substituted, deleted, and/or added and (ii) a pantothenate kinase activity.
(2) A polypeptide (C) or (D):
   (C) a polypeptide containing an amino acid sequence represented by SEQ ID NO: 4; or
   (D) a polypeptide having (i) an amino acid sequence, represented by SEQ ID NO: 4, one or more amino acids of which are substituted, deleted, and/or added and (ii) a pantothenate kinase activity.
(3) Isolated DNA (a) or (b):
   (a) DNA containing a base sequence represented by SEQ ID NO: 3; or
   (b) DNA which, under stringent conditions, hybridizes with DNA consisting of a base sequence complementary to a base sequence represented by SEQ ID NO: 3, and which encodes a polypeptide having a pantothenate kinase activity.
(4) Isolated DNA (c) or (d):
   (c) DNA containing a base sequence represented by SEQ ID NO: 5; or
   (d) DNA which, under stringent conditions, hybridizes with DNA consisting of a base sequence complementary to a base sequence represented by SEQ ID NO: 5, and which encodes a polypeptide having a pantothenate kinase activity.

The present invention includes (i) a recombinant vector containing the DNA and (ii) a transformant obtained by transforming a host cell with the recombinant vector.

Further, the present invention includes a method for preparing a pantothenic acid derivative (e.g., phosphopantothenic acid, phosphopantithenoylcysteine, phosphopantheteine, dephosphocoenzyme A, or coenzyme A) with use of (i) the polypeptide or (ii) the transformant having the DNA or the recombinant vector introduced therein. Furthermore, the present invention includes a method for preparing an oxidized pantothenic acid derivative that is obtained by oxidizing the pantothenic acid derivative.

### EFFECTS OF THE INVENTION

The use of a pantothenate kinase gene obtained by the present invention makes it possible to reinforce the rate-limiting biosynthetic enzymes. This makes it possible to efficiently produce pantothenic acid derivatives such as coenzyme A.

### BRIEF DESCRIPTION OF DRAWINGS

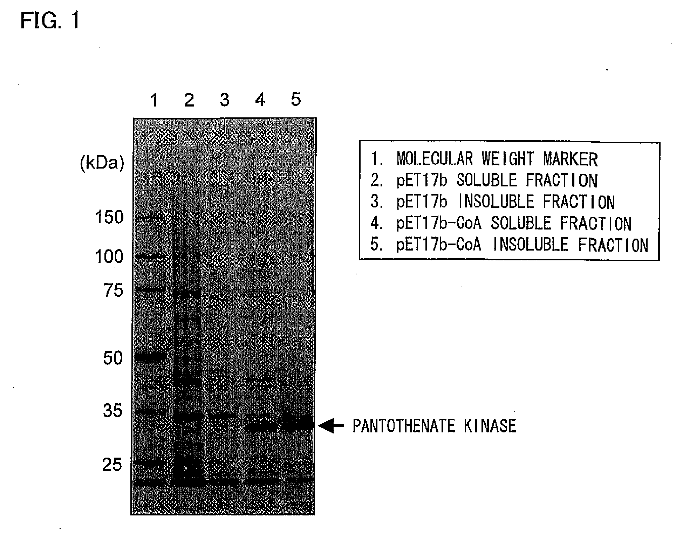

Fig. 1 shows a result of SDS-PAGE analysis of pantothenate kinase highly expressed in *Escherichia coli.*

### BEST MODE FOR CARRYING OUT THE INVENTION

### 1. Pantothenic Acid Derivative

The term "pantothenic acid derivative" used in this specification encompasses not only coenzyme A but also phosphopantothenic acid, phosphopantothenoylcysteine, phosphopantheteine, and dephosphocoenzyme A. In the following, those pantothenic acid derivatives are referred to as "pantothenic acid derivatives".

### 2. Pantothenate Kinase and CoaA-like Gene

In an aspect, the present invention provides the following polypeptide (A) or (B):
(A) a polypeptide containing an amino acid sequence represented by SEQ ID NO: 2; or
(B) a polypeptide having (i) an amino acid sequence, represented by SEQ ID NO: 2, one or more amino acids of which are substituted, deleted, and/or added and (ii) a pantothenate kinase activity.
   In another aspect, the present invention provides the following polypeptide (C) or (D):
(C) a polypeptide containing an amino acid sequence represented by SEQ ID NO: 4; or
(D) a polypeptide having (i) an amino acid sequence, represented by SEQ ID NO: 4, one or more amino acids of which are substituted, deleted, and/or added and (ii) a pantothenate kinase activity.

In another aspect, the present invention provides the following isolated DNA (a) or (b):
(a) DNA containing a base sequence represented by SEQ ID NO: 3; or
(b) DNA which, under stringent conditions, hybridizes with DNA consisting of a base sequence complementary to a base sequence represented by SEQ ID NO: 3, and which encodes a polypeptide having a pantothenate kinase activity.
   In another aspect, the present invention provides the following isolated DNA (c) or (d):
(c) DNA containing a base sequence represented by SEQ ID NO: 5; or
(d) DNA which, under stringent conditions, hybridizes with DNA consisting of a base sequence complementary to a base sequence represented by SEQ ID NO: 5, and which encodes a polypeptide having a pantothenate kinase activity.

In Examples, examples of the DNA of the present invention which encodes a polypeptide include, but are not limited to, DNA derived from *Corynebacterium ammoniagenes* NBRC 12612 and NBRC 12072, and include genes derived from other strains of *Corynebacterium ammoniagenes* that can be cloned by means well known to a person skilled in the art.

The *Corynebacterium ammoniagenes* NBRC 12612 and NBRC 12072 are preserved in the NITE Biological Resource Center of the National Institute of Technology and Evaluation (NBRC: 2-5-8 Kazusakamatari, Kisarazu City, Chiba 292-0818), and can be obtained from the organization.

In the following, embodiments concerning SEQ ID NO: 3 or SEQ ID NO: 5 in this aspect are referred to as "CoaA-like genes".

The term "pantothenate kinase" as used in this specification refers to an enzyme capable of acting as a catalyst for the first step in the biosynthesis of coenzyme A from pantothenic acid. The pantothenate kinase has been known since a long time ago to exist in most organisms, and a gene therefor has already been acquired from a large number of organisms. Specifically, the pantothenate kinase as an example of the present invention is an enzyme which can act as a catalyst for reactions by which D-phosphopantothenic acid, D-phosphopantothenoylcysteine, and D-phosphopantheteine are generated from D-pantothenic acid, D-pantothenoylcysteine, and D-pantheteine, respectively.

Whether or not a polypeptide encoded by a gene has such an activity of pantothenate kinase (an pantothenate kinase activity) can be directly measured, for example, by an assay method devised by Shimizu et al. (Shimizu S., et al., Agric. Biol. Chem., 37, 2863-2870 (1973)). Alternatively, for example, if an increase in generation of a pantothenic acid derivative can be confirmed by transforming a gene into a host microorganism, it can be confirmed that the polypeptide encoded by the gene has the pantothenate kinase activity not only *in vitro* but also *in vivo.*

Furthermore, the polypeptide (pantothenate kinase) of the present invention includes a polypeptide consisting of an amino acid sequence represented by SEQ ID NO: 2 or SEQ ID NO: 4 or a polypeptide substantially identical to the polypeptide.

The "polypeptide substantially identical" includes a case, for example, where the polypeptide has amino acid sequence identity of not less than 70% or not less that 80%, preferably not less than 85%, more preferably not less than 90%, or most preferably not less than 99%, with the amino acid sequence represented by SEQ ID NO: 2 or SEQ ID NO: 4, and has a function equivalent to that of the polypeptide consisting of the amino acid sequence represented by SEQ ID NO: 2 or SEQ ID NO: 4. In addition, the "polypeptide substantially identical" includes a case where the polypeptide contains an amino acid sequence which forms an active site of a polypeptide of Examples of the present invention and which is estimated to characterize a predisposition of the polypeptide is contained.

The amino acid sequence "identity" is determined by making a comparison between two sequences optimally aligned across the whole region of a sequence that is to serve as a target of comparison. The sequence that is to serve as a target of comparison may have an addition or a deletion (e.g., a gap) when optimally aligned. The amino acid sequence identity can be calculated based on the value of Identity with respect to the whole sequence, for example, in cases where the two amino acid sequences are compared and analyzed with use of a homology searching program BLAST (Altschul SF. et al, Nucleic Acids Res. (1997) 25:3389-3402). One or more amino acids of the amino acid sequence can be deleted, substituted, inserted, or added by a method well known to a person skilled in the art. Examples of such a method include site-directed mutagenesis.

Furthermore, the DNA of the present invention only needs to be DNA that encodes such a polypeptide of the present invention as described above. Specifically, the DNA of the present invention may be nucleic acid represented by SEQ ID NO: 3 or SEQ ID NO: 5, or may be nucleic acid, represented by SEQ ID NO: 3 or SEQ ID NO: 5, which has a base sequence, one or more bases of which are deleted, substituted, inserted, or added.

Further, the DNA of the present invention only needs to have base sequence identity of not less than 60% or not less than 70%, preferably not less than 80%, more preferably not less than 90%, or more preferably not less than 99%, with a base sequence represented by SEQ ID NO: 3 or SEQ ID NO: 5. The technical significance of the base sequence "identity" is the same as the contents explained as the amino acid sequence "identity".

The term "stringent conditions for hybridization" is not particularly limited as long as a base sequence contained in a test gene hybridizes with a base sequence contained in a gene having a function substantially equivalent to that of the test gene, but preferably refers to such conditions that hybridization is performed at approximately 50°C in a solution containing approximately 5×SSC (composition of 1 xSSC: 0.15 M NaCl, 0.015 M sodium citrate, pH 7.0), approximately 0.5% sodium dodecyl sulfate (SDS), and approximately 10 µg/ml of denatured sonicated salmon sperm DNA and then washing is performed at 50°C in approximately 2×SSC and approximately 0.1% of SDS.

More desirably, the term "stringent conditions for hybridization" refers to such conditions that hybridization is performed under the same conditions as described above and then washing is performed at 50°C in approximately 1xSSC and approximately 0.1% SDS. More desirably, the term "stringent conditions for hybridization" refers to such conditions that hybridization is performed under the same conditions as described above and then washing is performed at 50°C in approximately 0.5×SSC and approximately 0.1% SDS. More preferably, the term "stringent conditions for hybridization" refers to such conditions that hybridization is performed under the same conditions as described above and then washing is performed at 50°C in approximately 0.1 ×SSC and approximately 0.1% SDS. Even more preferably, the term "stringent conditions for hybridization" refers to such conditions that hybridization is performed under the same conditions as described above and then washing is performed at 65°C in approximately 0.1×SSC and approximately 0.1% SDS.

Needless to say, the conditions for hybridization can vary depending on the length of a nucleotide chain, the sequences, and different environmental parameters. A longer sequence hybridizes specifically at a higher temperature. A detailed guide to hybridization of nucleic acids is found, for example, in Tijssen (1993) Laboratory Techniques in Biochemistry and Molecular Biology--Hybridization with Nucleic Acid Probes part II chapter 2 "Overview of principles of hybridization and the strategy of nucleic acid probe assay" Elsvier, New York.

The polypeptide or the DNA is obtained by any method, but can be produced according to methods described in Examples.

### 3. Vector and Host

Usable examples of the vector mentioned herein include a plasmid vector and a phage vector. However, it is preferable that the vector have not only a drug resistance gene such as an appropriate antibiotic substance but also a restriction enzyme cleavage site into which foreign genes are to be incorporated and a promoter sequence located upstream of the site.

Usable examples of the vector are commercially available from Takara Bio Inc. and Stratagene. In an *Escherichia coli* system, a general-purpose pUC or pSTV vector having a promoter can be used. In a *Corynebacterium* bacterial system, a mutant gene can be incorporated with use of an *Escherichia coli* pHSG vector, or a *Corynebacterium* vector can be used.

Usable examples of the host mentioned herein include any host capable of establishing a method for performing transformation with use of a vector that can express an introduced gene incorporated thereinto.

Usable examples of the host include: bacteria such as *Escherichia coli, Bacillus subtilis, Corynebacterium,* and Rhizobium; actinomycetes such as Streptomyces; yeast such as *Saccharomyces cerevisiae, Schizosaccharomyces pombe,* and *Pichia pastolis;* fungi such as *Aspergillus;* animal cells; and plant cells.

In terms of practicality concerning substance production, it is preferable that the host be a microorganism such as bacteria selected from the group consisting of *Escherichia coli* and *Corynebacterium* bacteria.

### 4. Uses for Genes and the Like

Examples of uses for the genes, found by the inventors, which will be described later as Examples are as follows. First, such a gene is coupled with an appropriated plasmid vector, and then is transformed into a host such as *Escherichia coli* or *Corynebacterium* bacteria. The transformant thus obtained is cultured in culture medium containing a source of necessary nutrients. After the culture, a microorganism cell is recovered by centrifugation or filtration. Thus, a pantothenic acid derivative contained in the microorganism cell can be acquired by extraction or by crushing the microorganism cell. In addition, coenzyme A can be generated by using a reaction system obtained by suspending a microorganism cell in a mixture of (i) pantothenic acid, L-cysteine, and adenosine monophosphate (hereinafter abbreviated as "AMP") each serving as a substrate and (ii) a buffer solution containing glucose, a surfactant, and the like, and then by suspending the substrate and the microorganism cell so that they react with each other.

The pantothenic acid derivative thus produced in the reaction liquid is collected, and then is purified by an appropriate method such as a publicly-known purification technique. This makes it possible to obtain the target pantothenic acid derivative. The isolation of the pantothenic acid derivative from the reaction liquid can be performed, for example, with use of active carbon, florisil, alumina, silicate, powdered filter paper, porous synthetic polymer carriers (e.g., Amberlite IR120 (Rohm and Haas Company) and DIAION HP-20 (Mitsubishi Chemical Corporation)), various ion-exchange resins (e.g., DOWEX 1X2 (Acros Organics), Amberlite IRC-50 (Rohm and Hass Company), DEAE Cellose, DEAE Cephalose CL-6B (Amersham Biosciences K.K.)), gel filtration carriers (e.g., Sephadex G-25, Sephadex LH-20 (Amersham Biosciences K.K.), BIO-GEL P-2 (Bio-Rad Laboratories, Inc.)), or various affinity carriers. These carriers can be used alone or in combination as a column chromatography.

Further, it is conceivable that production is further increased by simultaneously introducing other genes involved in the biosynthesis of coenzyme A. A known enzyme gene involved in the biosynthesis of coenzyme A can be acquired by PCR with use of its own chromosome DNA based on DNA-sequence information that can be acquired from a publicly-known database (e.g., GenBank Database). Further, as with the obtainment of pantothenate kinase genes shown in Examples of the present invention, an unknown gene can be acquired by PCR with use of its affinity with an already acquired biosynthetic gene of another biologic origin. Preferably, by designing a PCR primer so that a sequence obtained by adding appropriate restriction enzyme cleavage points to both ends of the gene, and then by acquiring the sequence by PCR with use of the PCR primer, insertion into a vector is facilitated.

In order to express a biosynthetic enzyme gene that can be obtained by the above methods, a producing strain is prepared as follows. The aforementioned vector is cleaved with appropriate restriction enzymes. The vector is ligated with a biosynthetic enzyme gene cleaved with the same restriction enzymes. And then, the expression vector is transformed into *Escherichia coli* or *Corynebacterium* bacteria that serves as a host.

Recombinant producing bacteria can be cultured in the following manner. *Escherichia coli* can be cultured in 2YT culture medium or in L culture medium, and *Corynebacterium* bacteria can be cultured in B culture medium (obtained by dissolving 10g of glucose, 15 g of peptone, 3g of K₂HPO₄, 2 g of NaCl, 0.2 g of MgSO₄-7H₂O, and 1 g of yeast extract in 1 L of deionized water (hereinafter marked down as "/1L"), (pH 7.0)) or in G culture medium (150 g of glucose, 10 g of KH₂PO₄, 10 g of K₂HPO₄, 10 g of MgSO₄·7H₂O, 0.1 g of CaCl₂·2H₂O, 0.02 g of FeSO₄·7H₂O, 0.01 g of ZnSO₄·7H₂O, 0.02 g of MnSO₄·6H₂O, 0.02 g of L-cysteine, 0.005 g of thiamine hydrochloride, 0.015 g of β-alanine, 0.1 mg of biotin, 3 g of urea, 15 g of corn steep liquor, and 1 g of sodium glutamate/ 1L, pH 6.8). From the culture fluid after culture, microorganism cells are collected. The microorganism cells can be used for a reaction by which a pantothenic acid derivative is generated.

As described above, the present invention relates to a gene that can be used for advantageously producing a pantothenic acid derivative by a host organism (e.g., microorganism), a transformant having the gene introduced therein, and a method for preparing a pantothenic acid derivative with use of the transformant, and therefore can be advantageously used for producing a pantothenic acid derivative.

### 5. Preparation of an Oxidized Pantothenic Acid Derivative

According to the present invention, an oxidized pantothenic acid derivative can be prepared by oxidizing a pantothenic acid derivative prepared as described above. The term "oxidized pantothenic acid derivative" here refers to a compound obtained by forming a S-S bond by oxidizing a pantothenic acid derivative such as coenzyme A, dephosphocoenzyme A, phosphopantheteine, phosphopantothenoylcysteine, or pantheteine and a compound represented by R-SH (R being an organic group). Examples of the compound represented by R-SH include the pantothenic acid derivative, cysteine, cysteamine, and glutathione. That is, the oxidized pantothenic acid derivative encompasses: a compound (dimer) obtained by oxidizing a pantothenic acid derivative of the same kind; a compound obtained by oxidizing two different kinds of pantothenic acid derivative; a compound obtained by oxidizing a pantothenic acid derivative and cysteine, cysteamine, or glutathione; and other compounds.

The oxidization can be performed by a chemical method, a biochemical method using an enzyme or the like, or other methods, and is not particularly limited as long as the pantothenic acid derivative is not decomposed. However, the oxidization is easily performed by a method using an oxidizing agent. Examples of such a method include a method using an oxidizing agent such as hydrogen peroxide or potassium iodide.

### EXAMPLES

The present invention will be explained more in detail below with reference to Examples. However, the present invention is not limited to Examples. Basic operations for the following genetic recombination were performed according to methods described, for example, in Molecular Cloning, A Laboratory Manual, Third Edition, Cold Spring Harbor Laboratory (2001) and Methods in Enzymology, vol. 194 (1991). Further, in cases where a commercially-available kit was used, the attached instruction manual was followed.

### (Example 1) Obtainment and Confirmation of CoaA Genes

Genomic DNA of *Corynebacterium ammoniagenes* NBRC 12612 was prepared with use of a DNA Tissue Kit (QIAGEN).

Respective base sequences of a pantothenate kinase (CoaA) gene derived from *Corynebacterium efficiens,* a CoaA gene derived from *Corynebacterium glutamicum,* and a CoaA gene derived from *Corynebacterium diphtheriae* were compared. Attention was focused on sequences of highly conserved regions, and a mix primer was designed to contain those sequences of the highly conserved regions (Primer-1:5'-TAYCTNCCRCTGTCSCGTCTSAT-3' (SEQ ID NO: 6), Primer-2:5'-GCKCGMACYCGRGTGGGMAGRAT-3' (SEQ ID NO: 7) (where Y represents a sequence synthesized with use of a substrate mixture of C and T; N represents a sequence synthesized with use of a substrate mixture of A, C, G, and T; R represents a sequence synthesized with use of a substrate mixture of A and G; S represents a sequence synthesized with use of a substrate mixture of C and G, K represents a sequence synthesized with use of a substrate mixture of G and T; and M represents a sequence synthesized with use of a substrate mixture of A and C)). This mix primer was used to amplify a CoaA gene fragment from genomic DNA of *Corynebacterium ammoniagenes* NBRC 12612 by PCR. (Into 100 µL of reaction liquid, 2.5 units of Ex Taq HS (Takara Bio Inc.), 1× buffer included therewith, 0.2 mM of each dNTP, 80 pmol of each of Primer-1 and Primer-2, and 100 ng of template DNA were added. A hot-start technique was performed. Denaturization was performed at 98°C for 30 seconds. Thereafter, a cycle of 98°C for 10 seconds, 55°C for 30 seconds, and 72°C for 1 minute was repeated 30 times.) This resulted in a specifically amplified fragment of approximately 700 bp. The fragment thus obtained was cloned with pT7Blue T-Vector (Takara Bio Inc.), and a base sequence thereof was determined.

An outward primer was designed based on the determined 661-bp base sequence (Primer-3:5'-GGTGGATTTGCCCACGGCAA-3' (SEQ ID NO: 8), Primer-4:5'-GCCGATATGAATGACGAGCA-3' (SEQ ID NO: 9)), and inverse PCR was performed by using, as a template, a DNA fragment obtained by digesting the genomic DNA of *Corynebacterium ammoniagenes* NBRC 12612 with various restriction enzymes and then subjected to self ring closure. (Into 100 µL of reaction liquid, 2.5 units of Ex Taq HS, 1× buffer included therewith, 0.2 mM of each dNTP, 80 pmol of each of Primer-3 and Primer-4, and 100 ng of template DNA were added. A hot-start technique was performed. Denaturization was performed at 98°C for 30 seconds. Thereafter, a cycle of 98°C for 10 seconds, 55°C for 30 seconds, and 72°C for 2 minutes was repeated 30 times.) As a result, a 1.0-kbp specifically amplified fragment was obtained by the inverse PCR in which the DNA fragment obtained by digestion with NcoI and subjected to self ring closure was used as a template.

A base sequence of the fragment thus obtained was determined, and was found to contain all protein-coding portions (SEQ ID NO: 3) of the CoaA genes.

### (Example 2) Preparation of Escherichia coli Expression Vector

As a primer to acquire an encoding portion of a CoaA gene derived from *Corynebacterium,* Primer-5:5'-GGAATTCCATATGGCGCGGAACTCCGATTT-3' (SEQ ID NO: 10) and Primer-6:5'-CGCGGATCCCTAGAGCTTGCGCATCCGGA-3' (SEQ ID NO: 11) were synthesized. PCR was performed by using chromosome DNA of *Corynebacterium ammoniagenes* NBRC 12612 as a template. The obtained DNA fragment was cleaved with Ndel and BamHI, and then was ligated with pUCNde cleaved with those restriction enzymes. This resulted in a recombinant vector pUC-CoaA. The recombinant vector was used to transform *Escherichia coli* DH5a into a recombinant *Escherichia coli.* The recombinant *Escherichia coli* thus obtained is indicated as *"Escherichia coli* DH5ct/pUC-CoaA", and is deposited as Depository No. FERM BP-10541 at the International Patent Organism Depository (IPOD: AIST Tsukuba Central 6, 1-1, Higashi 1-chome., Tsukuba-shi, Ibaraki-ken 305-8566 Japan) of the National Institute of Advanced Industrial Science and Technology (Deposited on February 27, 2006: The strain originally deposited domestically on March 4, 2005 was later deposited internationally based on the Budapest Treaty).

### (Example 3) Evaluation of CoaA Gene Expression in Escherichia coli

A strain of *Corynebacterium ammoniagenes* NBRC 12612 was inoculated into culture medium (pH 7.0) containing 1% glucose, 1.5% peptone, 0.1% yeast extract, 0.3% K₂HPO₄, 0.2% NaCl, and 0.02% MgSO₄-7H₂O, and then was cultured at 28°C for 24 hours. The resulting product was used as a seed bacterial culture solution. Next, the above seed bacterial culture solution was inoculated by 10% into culture medium (pH 6.8) having a composition of 10% glucose, 1.5% urea (separately sterilized), 1% KH₂PO₄, 1% K₂HPO₄ 1% MgSO₄-7H₂O, 1% yeast extract, 0.5% peptone, 0.05% sodium L-glutamate, 0.05% L-serine, 0.05% isoleucine, and 0.005% β-alanine, and then was cultured with shaking at 28°C for approximately 72 hours. The microorganism cells thus cultured were collected by centrifugation, and were used for synthetic culture of coenzyme A.

Meanwhile, the recombinant *Escherichia coli* obtained in Example 2 or *Escherichia coli* DH5a serving as a host was precultured at 37°C in LB culture medium (containing 50 µl/ml of ampicillin in case of the recombinant *Escherichia coli*). Thereafter, the preculture fluid was inoculated by 1 % into LB culture medium (containing 50 µl/ml of ampicillin in case of the recombinant *Escherichia coli*)*,* and then was shake-cultured at 37°C for 24 hours. The microorganism cells thus cultured were collected by centrifugation, and were used for synthetic culture of coenzyme A.

Into a flask having a capacity of 500 ml, 20 ml of reaction liquid containing 15% glucose, 2% KH₂PO₄, 0.5% MgSO₄·7H₂O, 0.355% calcium pantothenate, 1.8% cysteine, 0.6% AMP·Na, 1% xylene, 0.4% polyoxyethylene stearylamine S-220, 100 g/l of a wet microorganism cell of *Corynebacterium ammoniagenes* NBRC 12612, and 50 g/l of a wet microorganism cell of the recombinant *Escherichia coli* or host *Escherichia coli* was poured. The concentration of CoA accumulated on the supernatant of culture fluid that had been shaken at 28°C for 96 hours was measured. As a result, in cases where the recombinant *Escherichia coli* was used, a value of 0.073 g/ l (CoA concentration per liter of culture fluid) was obtained, which indicated approximately 1.8 times as high a level of productivity as in cases where the host *Escherichia coli* was used.

### (Example 4) Expression of CoaA Gene Activity by Recombinant Escherichia coli

The CoaA gene obtained in Example 2 was inserted into pET-17b with use of an Ndel-BamHI restriction enzyme site, and was transformed into *Escherichia coli* Rosetta 2 (DE3) pLysS. The transformed strain was precultured in 2 ml of L culture medium containing 50 µg/ml of ampicillin and 30 µg/ml of chloramphenicol. The transformed strain thus precultured was inoculated by 1 % into 100 ml of main culture medium (NZCYM culture medium containing 50 µg/ ml of ampicillin and 30 µg/ ml of chloramphenicol), and then was cultured aerobically for 4 hours. Thereafter, IPTG was added for induction, and culture was performed aerobically for 3 hours. Microorganism cells were collected from the culture fluid by centrifugation, and were disrupted by ultrasonication for preparing cell-free extract. Each of the soluble fraction and insoluble fraction of the cell-free extract was analyzed by SDS-PAGE. As a result, it was found that the transformed strain clearly shows an increase in generation of pantothenate kinase (see Fig. 1).

### (Example 5) Expression of CoaA Gene Activity in Corynebacterium ammoniagenes

First, for the purpose of preparing an *Escherichia coli-Corynebacterium* shuttle vector, a PCR-amplified fragment (amplified with use of Primer-7:5'-GAACGTTCGTTATAATGGTG-3' (SEQ ID NO: 12) and Primer- 8:5'-TGTGTTTCTC CC GTGTC CGG- 3' (SEQ ID NO: 13)) of a *Corynebacterium* vector pCG11 was ligated with a cloning site of an *Escherichia coli* vector pGEM-T Easy. This resulted in an *Escherichia coli-Corynebacterium* shuttle vector pGEM-CG11. The CoaA gene obtained in Example 2 was inserted into the pG EM-CG 11 with use of a restriction enzyme PstI site, and Corynebacterium ammoniagenes NBRC 12612 was transformed by electroporation. The transformed strain was precultured in 5 ml of B culture medium containing 10 µg/ml of streptomycin. The transformed strain thus precultured was inoculated into 50 ml of main culture medium, and then was cultured aerobically for 3 days. Microorganism cells were collected from the culture fluid by centrifugation, and were disrupted by ultrasonication for preparing cell-free extract. Measurement of pantothenate kinase activity was performed by a microbiological method of Shimizu et al. for quantitatively determining the amount of pantothenic acids (Agric. Biol. Chem., 37, 2863-2870 (1973)). As a result, it was confirmed that the transformed strain has a specific activity of 1.77 units/mg protein, i.e., attains 10 times as high a level of expression as a wild strain, which has a specific activity of 0.16 units/mg protein.

### (Example 6) Obtainment and Confirmation of CoaA Genes of Corynebacterium ammoniagenes NBRC 12072

Genomic DNA of *Corynebacterium ammoniagenes* NBRC 12072 was prepared with use of a DNA Tissue Kit (QIAGEN). PCR was performed in the same manner as in Example 1 with use of the primer of Example 1 and the genomic DNA of *Corynebacterium ammoniagenes* NBRC 12072. A base sequence of the fragment thus obtained was determined, and was found to contain all protein-coding regions (SEQ ID NO: 5) of the CoaA genes.

## Claims

1. A polypeptide (A) or (B):
(A) a polypeptide containing an amino acid sequence represented by SEQ ID NO: 2; or
(B) a polypeptide having (i) an amino acid sequence, represented by SEQ ID NO: 2, one or more amino acids of which are substituted, deleted, and/or added and (ii) a pantothenate kinase activity.

2. A polypeptide (C) or (D):
(C) a polypeptide containing an amino acid sequence represented by SEQ ID NO: 4; or
(D) a polypeptide having (i) an amino acid sequence, represented by SEQ ID NO: 4, one or more amino acids of which are substituted, deleted, and/or added and (ii) a pantothenate kinase activity.

3. Isolated DNA (a) or (b):
(a) DNA containing a base sequence represented by SEQ ID NO: 3; or
(b) DNA which, under stringent conditions, hybridizes with DNA consisting of a base sequence complementary to a base sequence represented by SEQ ID NO: 3, and which encodes a polypeptide having a pantothenate kinase activity.

4. Isolated DNA (c) or (d):
(c) DNA containing a base sequence represented by SEQ ID NO: 5; or
(d) DNA which, under stringent conditions, hybridizes with DNA consisting of a base sequence complementary to a base sequence represented by SEQ ID NO: 5, and which encodes a polypeptide having a pantothenate kinase activity.

5. A recombinant vector containing nucleic acid as set forth in claim 3 or 4.

6. A transformant obtained by transforming a host cell with DNA as set forth in claim 3 or 4 or with a recombinant vector as set forth in claim 5.

7. The transformant as set forth in claim 6, wherein the host is a microorganism.

8. The transformant as set forth in claim 6, wherein the host is *Corynebacterium ammoniagenes.*

9. The transformant as set forth in claim 6, wherein the host is *Escherichia coli.*

10. A method for preparing a pantothenic acid derivative, comprising producing, with use of a polypeptide as set forth in claim 1 or 2 or with use of a transformant as set forth in claim 6, at least one type of pantothenic acid derivative selected from the group consisting of phosphopantothenic acid, phosphopantithenoylcysteine, phosphopantheteine, dephosphocoenzyme A, and coenzyme A.

11. A method for preparing an oxidized pantothenic acid derivative, comprising producing an oxidized pantothenic acid derivative by oxidizing a pantothenic acid derivative obtained by a method as set forth in claim 10.


SEQUENCE LISTING

<110>  KANEKA CORPORATION

<120>  NOVEL PANTOTHENATE KINASE GENE AND USES THEREFOR

<130>  P23633

<150>  JP2005-094183
<151>  2005-03-29

<160>  13

<170>  PatentIn version 3.1

<210>  1
<211>  4028
<212>  DNA
<213>  Corynebacterium ammoniagenes

<220>
<221>  CDS
<222>  (1286)..(2209)
<223>

<400>  1
caatgcgcag accggaggtc accattggtg gacgaggatc attaggaacc gcattgcggt      60

tgacggtgat accaacctcg tgcaggaggt cttcggcctg ctggccatcc atctccgagt     120

tgcgcaggtc aaccaaaacc aggtgcacgt cggtgccgcc ggtgagtacg tcaacgcctg     180

cggccttagc tgcggacgag gtcaagcgct cagccaagat ctgtgcgcct tcgacggtgc     240

gagcctgacg ctcacggaaa tcttccgacg cggcaatctt catcgcaatc gccttcgctg     300

ccaccacgtg catcaaagga ccaccctgct ggcctgggaa gaccgcagag ttgagtttct     360

tggcgtagtc ctgcttggcc aagatcatgc cggagcgtgg gccaccgagg gtcttgtgca     420

cggtggtgga gactacatca gagtgtggca ctgggttcgg atacagatcc gctgcaacca     480

gaccagcgaa gtgagccata tcggtccaca agtaagcgcc aacttcatcg gcgatggaac     540

ggaatgcttc aaaatccagg gtgcgcggat atgccgacca acccgcgatg atcacctgtg     600

gcttttcagc gattgcctgc tcgcgcagac ggtccatatc cagacggcta gtcttttcat     660

ccaccgtata agcagctact tcgtacagct tgccggagaa gttcagcttc atgccgtggg     720

tcaggtgtcc accatgatcc aacgaaagac ccagaatctt atcgccgggg ttgataagtg     780

atgccaacac cgcagcgttt gcctgtgcac ccgagtgggg ctgaacattg gcaaagtccg     840

cgccgaagag agactttgcg cggtcacgtg ccaagtcttc gatgatgtca acatgctcac     900

aaccaccgta gtaacgacgg ccagggtatc cctcagcgta cttgttggta agtaccgagc     960

cttgtgcctg caaaactgcg cgaggtacaa agttctccga agcaatcatt tccagggtgt    1020

cgcgctgacg tgcgatttca ccgttgatgg ctgcataaac ctcagggtct agatcgcgca    1080

tttcctgata acgcgcatcg gaagaattct gagtagtcat atgactatat attcccttcg    1140

tcgaagtggg gatgaagcaa tacaccacac atgatagcca agggcacccc ggatcagaga    1200

actttcccct attaaaattt tccggcggca gcctcagatg tgggttaatt tcctcgtaac    1260

cttgcatagt gacacaatgg aggcc atg gcg cgg aac tcc gat tta agc ccc 1312
Met Ala Arg Asn Ser Asp Leu Ser Pro
1               5

tac ctt gat ttc gac cgt gac acc tgg cgt gga ctt cgc aac tcc atg 1360
Tyr Leu Asp Phe Asp Arg Asp Thr Trp Arg Gly Leu Arg Asn Ser Met
10                  15                  20                  25

ccg cag gtt ctg acg gaa gcc gaa gtt aag aag ctc gcg ggc att ggt 1408
Pro Gln Val Leu Thr Glu Ala Glu Val Lys Lys Leu Ala Gly Ile Gly
                30                  35                  40

gaa aac atc aac ctc gaa gag gtc gca gat gtc tac ctt ccg ctt tcc 1456
Glu Asn Ile Asn Leu Glu Glu Val Ala Asp Val Tyr Leu Pro Leu Ser
            45                  50                  55

cgt ctt atc cac atg cag gtt gcc gcg cga cag gcg ttg acc caa gcc 1504
Arg Leu Ile His Met Gln Val Ala Ala Arg Gln Ala Leu Thr Gln Ala
        60                  65                  70

acc gag cag ttc tta ggt aac ccg ccg agt cac gtg cct ttt gtc atc 1552
Thr Glu Gln Phe Leu Gly Asn Pro Pro Ser His Val Pro Phe Val Ile
    75                  80                  85

ggc gtg gcg ggt tct gtt gcc gtg ggc aaa tcc acc act gca cgt ttg 1600
Gly Val Ala Gly Ser Val Ala Val Gly Lys Ser Thr Thr Ala Arg Leu
90                  95                  100                 105

ctt caa gtc ctt ttg gaa cgc tgg gat acc cac ccc aaa gtc gat ctc 1648
Leu Gln Val Leu Leu Glu Arg Trp Asp Thr His Pro Lys Val Asp Leu
                110                 115                 120

gtg aca acg gat ggc ttt ctc tac ccc act cgc att ttg caa gaa cgc 1696
Val Thr Thr Asp Gly Phe Leu Tyr Pro Thr Arg Ile Leu Gln Glu Arg
            125                 130                 135

ggg ctg atg tcg cgc aag ggc ttt ccc gaa tct tat gac cgc cgt tcg 1744
Gly Leu Met Ser Arg Lys Gly Phe Pro Glu Ser Tyr Asp Arg Arg Ser
        140                 145                 150

ctg atg cgc ttt gtc acg gat gtt aaa tcc ggc aag gaa gta gtc cgc 1792
Leu Met Arg Phe Val Thr Asp Val Lys Ser Gly Lys Glu Val Val Arg
    155                 160                 165

gca ccg ctg tac tcg cac gtg tcc tat gac atc gtt cca aat gaa tac 1840
Ala Pro Leu Tyr Ser His Val Ser Tyr Asp Ile Val Pro Asn Glu Tyr
170                 175                 180                 185

cag acg gtt aat cag ccc gat atc ttg att ctg gaa ggg ctc aat gtt 1888
Gln Thr Val Asn Gln Pro Asp Ile Leu Ile Leu Glu Gly Leu Asn Val
                190                 195                 200

ttg cag acc ggc cct acc ctg atg att tcg gat ctt ttt gat ttc tcg 1936
Leu Gln Thr Gly Pro Thr Leu Met Ile Ser Asp Leu Phe Asp Phe Ser
            205                 210                 215

gtc tac gtg gat gcg cgc gta gag acc atc gaa aag tgg tac atc aac 1984
Val Tyr Val Asp Ala Arg Val Glu Thr Ile Glu Lys Trp Tyr Ile Asn
        220                 225                 230

cgc ttc ctc gac ctg cgc cac act gcc ttc cgt gaa ccc gga gcg cac 2032
Arg Phe Leu Asp Leu Arg His Thr Ala Phe Arg Glu Pro Gly Ala His
    235                 240                 245

ttc gcg gcc tat gcc gat atg aat gac gag cag gct ttt aac cag gct 2080
Phe Ala Ala Tyr Ala Asp Met Asn Asp Glu Gln Ala Phe Asn Gln Ala
250                 255                 260                 265

cgt gaa att tgg cag tcg att aac ctg ccc aac ctc gtc gaa aat att     2128
Arg Glu Ile Trp Gln Ser Ile Asn Leu Pro Asn Leu Val Glu Asn Ile
            270                 275                 280

ttg ccc acg cgc gtg cgg gca tcg ctg gtg ttg cgc aaa ggc gca gac     2176
Leu Pro Thr Arg Val Arg Ala Ser Leu Val Leu Arg Lys Gly Ala Asp
        285                 290                 295

cac acc gtg gaa aaa gtc cgg atg cgc aag ctc taggctgggg tttcccgagc     2229
His Thr Val Glu Lys Val Arg Met Arg Lys Leu
    300                 305

tgaggcttcc cgccttgggc ttcctgcccg aaggcttcca gcctgtggcg cgtctatttt     2289

ccgaagcggc gggagcgctg ggagaaatcg cgcagggcgc ggaggaaatc aatcttgcgg     2349

aatgccggcc agtaggtgtc ggtaaaccag atttcagaat acgcggcttg ccacagcagg     2409

aagccggata gtcgctgttc gccggaggtg cggatgacga gatccgggtc cggcagcccc     2469

ttggtataca ggtgcttcga aatggaatcc acggtgatgc tctccaccaa ttcctctggc     2529

gttgcccccg cttcgacttc ttttttcagc atggccttca cggcgtccgc aatctcttga     2589

cggccgccgt aaccaaccgc gatattgacg atgaccccgt cattgtcccg ggtatcgtcc     2649

gctgccgaac acatcgcgtt tgtaacctca tccggcaata ggtccaggtg tccgaccagg     2709

cgcacctggc agttcaggtc gccatggctg aggtggtcca cgacctcggt gatgatatcg     2769

aataagaggc tgacctcatt tttcgaacgc ttgaggtttt ctgtcgacag cagatagatg     2829

gtgactactt ccacctcggt atcggcacac cacgagacca tctcgctaat cttttttgca     2889

ccttggcggt ggccgtggct gatatcttca aaccccgctt ccctagccca gcggcgattg     2949

ccgtcagcca tgactgcaac gtgcttgggc tgcggcttgc ctttgagctc ggagataaga     3009

cgggcttcat aggctgggta gagtaacgcg ggtagaagtc tcacaccagc attctatcgt     3069

gttgcgcggc gagtacgaac cgcagctgcc aactccgcca atagatccac ggtgtcgctg     3129

atatccatgc aggcatcggt gatggattgg ccataggtta acccctcaat tcccttctca     3189

tctaaggatt gcgcaccgcc ttcgaggaag gattccatca tgatgcccgc aatatgctca     3249

ttgccttcag caatttgctt agcgatgccc cgggttacct ctacctggcg cacatgatct     3309

tttccagagt tggcatggga ggcatcaatc atcatgcggg agtcatcgcc tagcttgtcg     3369

acgacctgcg ctaccgattc agcatcccag ttcggaccgt gggagccacc acgcaaaata     3429

atgtggcagt gctggttgcc cgcggtttct acgacggaag gctggccatc gtcagaggtt     3489

ccaaagaaga agtgcggcga tgctgctgcc gatgccgcgt gaatagccac gtccacgttg     3549

ccatcagtgc catttttgaa gccaatcggc atggacatcc ccgacgccag ctgacgatgc     3609

acctgggatt cggtggtacg agcgccgata gcgccccaag cgactgcatc agcgtagtac     3669

tgaggtgagt tcggctctaa gaactcgcac gcggctggca ggtctaggtt cacaatatcg     3729

gtgagcacct tgcgcgcggt gcgcaggccg gaaggaatat cgaaggactg atccaagtgt     3789

gggtcattga taagtccctt ccagcccacg gtggtgcgag gcttttcaaa gtacacgcgc     3849

ataaccacct tgaggtcctc ctccagcgcg tgggccagcg gagacaagcg gtttgcgtaa     3909

tccagcgcgg cgacgggatc gtgaatcgag cagggcccca cgaccactac gaggcggtcg 3969
tcttcaccag cgaagatatc ggcaatttcc tgacgatcct gctctacctt cgcggcttg 4028

<210> 2
<211> 308
<212> PRT
<213> Corynebacterium ammoniagenes

<400> 2

Met Ala Arg Asn Ser Asp Leu Ser Pro Tyr Leu Asp Phe Asp Arg Asp
1               5                   10                  15

Thr Trp Arg Gly Leu Arg Asn Ser Met Pro Gln Val Leu Thr Glu Ala
            20                  25                  30

Glu Val Lys Lys Leu Ala Gly Ile Gly Glu Asn Ile Asn Leu Glu Glu
        35                  40                  45

Val Ala Asp Val Tyr Leu Pro Leu Ser Arg Leu Ile His Met Gln Val
    50                  55                  60

Ala Ala Arg Gln Ala Leu Thr Gln Ala Thr Glu Gln Phe Leu Gly Asn
65                  70                  75                  80

Pro Pro Ser His Val Pro Phe Val Ile Gly Val Ala Gly Ser Val Ala
                85                  90                  95

Val Gly Lys Ser Thr Thr Ala Arg Leu Leu Gln Val Leu Leu Glu Arg
            100                 105                 110

Trp Asp Thr His Pro Lys Val Asp Leu Val Thr Thr Asp Gly Phe Leu
        115                 120                 125

Tyr Pro Thr Arg Ile Leu Gln Glu Arg Gly Leu Met Ser Arg Lys Gly
    130                 135                 140

Phe Pro Glu Ser Tyr Asp Arg Arg Ser Leu Met Arg Phe Val Thr Asp
145                 150                 155                 160

Val Lys Ser Gly Lys Glu Val Val Arg Ala Pro Leu Tyr Ser His Val
                165                 170                 175

Ser Tyr Asp Ile Val Pro Asn Glu Tyr Gln Thr Val Asn Gln Pro Asp
            180                 185                 190

Ile Leu Ile Leu Glu Gly Leu Asn Val Leu Gln Thr Gly Pro Thr Leu
        195                 200                 205

Met Ile Ser Asp Leu Phe Asp Phe Ser Val Tyr Val Asp Ala Arg Val
    210                 215                 220

Glu Thr Ile Glu Lys Trp Tyr Ile Asn Arg Phe Leu Asp Leu Arg His
225                 230                 235                 240

Thr Ala Phe Arg Glu Pro Gly Ala His Phe Ala Ala Tyr Ala Asp Met
                245                 250                 255

Asn Asp Glu Gln Ala Phe Asn Gln Ala Arg Glu Ile Trp Gln Ser Ile
            260                 265                 270

Asn Leu Pro Asn Leu Val Glu Asn Ile Leu Pro Thr Arg Val Arg Ala
        275                 280                 285

Ser Leu Val Leu Arg Lys Gly Ala Asp His Thr Val Glu Lys Val Arg
    290                 295                 300

Met Arg Lys Leu
305

<210>  3
<211>  927
<212>  DNA
<213>  Corynebacterium ammoniagenes

<400>  3
atggcgcgga actccgattt aagcccctac cttgatttcg accgtgacac ctggcgtgga      60

cttcgcaact ccatgccgca ggttctgacg gaagccgaag ttaagaagct cgcgggcatt     120

ggtgaaaaca tcaacctcga agaggtcgca gatgtctacc ttccgctttc ccgtcttatc     180

cacatgcagg ttgccgcgcg acaggcgttg acccaagcca ccgagcagtt cttaggtaac     240

ccgccgagtc acgtgccttt tgtcatcggc gtggcgggtt ctgttgccgt gggcaaatcc     300

accactgcac gtttgcttca agtccttttg gaacgctggg atacccaccc caaagtcgat     360

ctcgtgacaa cggatggctt tctctacccc actcgcattt tgcaagaacg cgggctgatg     420

tcgcgcaagg gctttcccga atcttatgac cgccgttcgc tgatgcgctt tgtcacggat     480

gttaaatccg gcaaggaagt agtccgcgca ccgctgtact cgcacgtgtc ctatgacatc     540

gttccaaatg aataccagac ggttaatcag cccgatatct tgattctgga agggctcaat     600

gttttgcaga ccggccctac cctgatgatt tcggatcttt ttgatttctc ggtctacgtg     660

gatgcgcgcg tagagaccat cgaaaagtgg tacatcaacc gcttcctcga cctgcgccac     720

actgccttcc gtgaacccgg agcgcacttc gcggcctatg ccgatatgaa tgacgagcag     780

gcttttaacc aggctcgtga aatttggcag tcgattaacc tgcccaacct cgtcgaaaat     840

attttgccca cgcgcgtgcg ggcatcgctg gtgttgcgca aaggcgcaga ccacaccgtg     900

gaaaaagtcc ggatgcgcaa gctctag                                         927

<210>  4
<211>  308
<212>  PRT
<213>  Corynebacterium ammoniagenes

<400> 4

Met Ala Arg Asn Ser Asp Leu Ser Pro Tyr Leu Asp Phe Asp Arg Asp
1               5                   10                  15

Thr Trp Arg Gly Leu Arg Lys Ser Met Pro Gln Val Leu Thr Glu Ser
            20                  25                  30

Glu Val Lys Lys Leu Ala Gly Ile Gly Glu Asn Ile Asn Leu Glu Glu
        35                  40                  45

Val Ala Asp Val Tyr Leu Pro Leu Ser Arg Leu Ile His Met Gln Val
    50                  55                  60

Ala Ala Arg Gln Ala Leu Thr Gln Ala Thr Glu Gln Phe Leu Gly Asn
65                  70                  75                  80

Pro Pro Ser His Val Pro Phe Val Ile Gly Val Ala Gly Ser Val Ala
                85                  90                  95

Val Gly Lys Ser Thr Thr Ala Arg Leu Leu Gln Val Leu Leu Glu Arg
            100                 105                 110

Trp Asp Thr His Pro Lys Val Asp Leu Val Thr Thr Asp Gly Phe Leu
        115                 120                 125

Tyr Pro Thr Arg Ile Leu Gln Glu Arg Gly Leu Met Ser Arg Lys Gly
    130                 135                 140

Phe Pro Glu Ser Tyr Asp Arg Arg Ala Leu Met Arg Phe Val Thr Asp
145                 150                 155                 160

Val Lys Ser Gly Lys Glu Val Val Arg Ala Pro Leu Tyr Ser His Val
                165                 170                 175

Ser Tyr Asp Ile Val Pro Asn Glu Tyr Gln Met Val His Gln Pro Asp
            180                 185                 190

Ile Leu Ile Leu Glu Gly Leu Asn Val Leu Gln Thr Gly Pro Thr Leu
        195                 200                 205

Met Ile Ser Asp Leu Phe Asp Phe Ser Val Tyr Val Asp Ala Arg Val
    210                 215                 220

Glu Thr Ile Glu Lys Trp Tyr Ile Asn Arg Phe Leu Gly Leu Arg His
225                 230                 235                 240

Thr Ala Phe Arg Glu Pro Gly Ala His Phe Ala Ala Tyr Ala Asp Met
                245                 250                 255

Asn Asp Glu Gln Ala Phe Asn Gln Ala Arg Glu Ile Trp Gln Ser Ile
            260                 265                 270

Asn Leu Pro Asn Leu Val Glu Asn Ile Leu Pro Thr Arg Val Arg Ala
        275                 280                 285

Ser Leu Val Leu Arg Lys Gly Glu Asp His Thr Val Glu Lys Val Arg
    290                 295                 300

Met Arg Lys Leu
305

<210>  5
<211>  924
<212>  DNA
<213>  Corynebacterium ammoniagenes

<400>  5
atggcgcgga actccgattt aagcccctac cttgatttcg accgcgatac ctggcgtgga      60

ctgcgtaagt ccatgccgca ggtattgact gaatccgaag tgaagaagct cgcgggcatt     120

ggtgaaaaca tcaacctcga agaggtggct gatgtctatc ttccgctttc ccgtctcatc     180

cacatgcagg tcgcagcgcg gcaagcacta actcaggcaa cagaacaatt cctgggaaac     240

ccgcccagcc acgtgccttt tgtcatcggt gtcgcaggtt ctgtcgcggt aggaaaatcc     300

acgacagcac gtttgctgca agttctacta gagcgctggg acactcaccc gaaggtcgac     360

ttggtcacga ccgacggatt cttatacccc acccggatct tgcaagagcg cggtttgatg     420

tcgcgcaaag gcttcccaga atcttatgac cgccgcgcgt taatgcgctt tgtcaccgac     480

gttaaatcag gaaaagaagt cgtccgcgct cccctgtact cacacgtgtc ttatgacatt     540

gtgcccaatg agtaccagat ggttcaccag cccgatattt taatcctcga aggcctcaac     600

gttttacaaa ccgggccaac gctgatgatt tcagatctct ttgatttctc ggtgtatgtg     660

gatgcgcggg tggaaaccat cgaaaagtgg tacatcaacc gcttcctcgg cttgcgccat     720

actgccttcc gtgagcccgg cgcacacttt gccgcttatg ccgatatgaa tgatgaacag     780

gcttttaatc aggcccgcga aatttggcaa tcaatcaact tgccgaatct ggtagagaat     840

attcttccta cccgcgtgcg cgcatcattg gtgctacgca aaggcgagga tcacacggtg     900

gaaaaagtcc gcatgcgcaa gctt                                            924

<210>  6
<211>  23
<212>  DNA
<213>  Artificial

<220>
<223>  Primer-1

<400>  6
tayctnccrc tgtcscgtct sat                                              23

<210>  7
<211>  23
<212>  DNA

<213>  Artificial

<220>
<223>  Primer-2

<400>  7
gckcgmacyc grgtgggmag rat                                              23


<210>  8
<211>  20
<212>  DNA
<213>  Artificial

<220>
<223>  Primer-3

<400>  8
ggtggatttg cccacggcaa                                                  20


<210>  9
<211>  20
<212>  DNA
<213>  Artificial

<220>
<223>  Primer-4

<400>  9
gccgatatga atgacgagca                                                  20


<210>  10
<211>  30
<212>  DNA
<213>  Artificial

<220>
<223>  Primer-5

<400>  10
ggaattccat atggcgcgga actccgattt                                       30


<210>  11
<211>  29
<212>  DNA
<213>  Artificial

<220>
<223>  Primer-6

<400>  11
cgcggatccc tagagcttgc gcatccgga                                        29


<210>  12
<211>  20
<212>  DNA
<213>  Artificial

<220>
<223>  Primer-7

<400>  12
gaacgttcgt tataatggtg                                                  20

<210>  13
<211>  20
<212>  DNA
<213>  Artificial

<220>
<223>  Primer-8

<400>  13
tgtgtttctc ccgtgtccgg                                                    20